# EUROPEAN PATENT APPLICATION

(11) **EP 0 614 987 A1**
(43) Date of publication of application: **14.09.1994**
(21) Application number: 94102038.0
(22) Date of filing: 10.02.1994
(51) Int. Cl.: C12Q 1/68, G01N 33/543

(54) **Flow-through membrane nucleic acid hybridization assay**

(30) Priority: 26.02.1993 US 26139
(71) Applicant: Becton Dickinson and Company, Franklin Lakes, New Jersey 07417-1880 (US)
(72) Inventor: Jurgensen, Stewart Russell, Raleigh, North Carolina 27615 (US); Donahue, Catherine Spargo, Cary, North Carolina 27511 (US); Shank, Daryl Dee, Durham, North Carolina 27712 (US)
(74) Representative: Rambelli, Paolo

(57) **Abstract**

A flow-through nucleic acid hybridization assay method employing a porous membrane of nylon activated for covalent attachment of proteins. The choice of materials and methods obviates the need for incubation of the membrane with the hybridization solution prior to initiating flow-through, resulting in a simpler and more rapid assay method.

### SEQUENCE LISTING

(1) GENERAL INFORMATION:
   (i) APPLICANT: Becton, Dickinson and Company
      (A) NAME: Becton,Dickinson and Company
      (B) STREET: One Becton Drive
      (C) CITY: Franklin Lakes
      (D) STATE: NJ
      (E) COUNTRY: USA
      (F) ZIP: 07417-1880
   (ii) TITLE OF INVENTION: Flow Through Membrane Nucleic Acid Hybridization Assay
   (iii) NUMBER OF SEQUENCES: 7
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (v) CURRENT APPLICATION DATA:
      (A) APPLICATION NUMBER: 94102038.0
      (B) FILING DATE: February 10, 1994
(2) INFORMATION FOR SEQ ID NO:1:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (v) FRAGMENT TYPE: internal
   (vi) ORIGINAL SOURCE:
      (A) ORGANISM: Chlamydia trachomatis
      (B) STRAIN: L2
   (xi) SEQUENCE DESCRIPTION: SEQ ID NO:1:
   TTCGTATTGC ACAGCCGAAG T 21
(2) INFORMATION FOR SEQ ID NO:2:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (v) FRAGMENT TYPE: internal
   (vi) ORIGINAL SOURCE:
      (A) ORGANISM: Chlamydia trachomatis
      (B) STRAIN: L2
   (xi) SEQUENCE DESCRIPTION: SEQ ID NO:2:
   GGAGATCCTT GCGATCCTTG C 21
(2) INFORMATION FOR SEQ ID NO:3:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (v) FRAGMENT TYPE: internal
   (vi) ORIGINAL SOURCE:
      (A) ORGANISM: Chlamydia trachomatis
      (B) STRAIN: L2
   (xi) SEQUENCE DESCRIPTION: SEQ ID NO:3:
   GTTGAGTTGT ATACAGATAC TACTTTTGC 29
(2) INFORMATION FOR SEQ ID NO:4:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (v) FRAGMENT TYPE: internal
   (vi) ORIGINAL SOURCE:
      (A) ORGANISM: Chlamydia trachomatis
      (B) STRAIN: L2
   (xi) SEQUENCE DESCRIPTION: SEQ ID NO:4:
   TTGGAGTGCT GGAGCTCGTG 20
(2) INFORMATION FOR SEQ ID NO:5:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (v) FRAGMENT TYPE: internal
   (vi) ORIGINAL SOURCE:
      (A) ORGANISM: Chlamydia trachomatis
      (B) STRAIN: L2
   (xi) SEQUENCE DESCRIPTION: SEQ ID NO:5:
   CAGCTTTGTG GGAATGTGGA TG 22
(2) INFORMATION FOR SEQ ID NO:6:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (v) FRAGMENT TYPE: internal
   (vi) ORIGINAL SOURCE:
      (A) ORGANISM: Chlamydia trachomatis
      (B) STRAIN: L2
   (xi) SEQUENCE DESCRIPTION: SEQ ID NO:6:
   AACAAGATGA AATCTAGAAA ATCTTG                                   26
(2) INFORMATION FOR SEQ ID NO:7:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (v) FRAGMENT TYPE: internal
   (vi) ORIGINAL SOURCE:
      (A) ORGANISM: Chlamydia trachomatis
      (B) STRAIN: L2
   (xi) SEQUENCE DESCRIPTION: SEQ ID NO:7:
   TTCGTATTGC ACAGCCGAAG T                                        21

## Description

### FIELD OF THE INVENTION

The present invention relates to membrane flow-through assays for nucleic acids, in particular nucleic acid sandwich hybridization assays.

### BACKGROUND OF THE INVENTION

Hybridization of complementary nucleic acid sequences is a well known means for identifying, detecting and isolating specific nucleic acids and nucleic acid sequences. Such hybridization methods form the basis of many current nucleic acid assays. The principle of hybridization assays is to expose two or more single stranded nucleic acids to each other, allow complementary sequences to hybridize, and then measure the amount of double stranded nucleic acid formed. The nucleic acid or sequence which is sought to be identified, detected or isolated is customarily called the target nucleic acid or target sequence.

A single stranded nucleic acid or sequence which is used to bind to the target nucleic acid to isolate, identify or detect is customarily called a probe. In some methods of nucleic acid hybridization, more than one probe may be used for different purposes. A probe often has a detectable label incorporated into the structure of the probe which facilitates identification of the target nucleic acid when the probe hybridizes to it. The label produces a signal which can be detected either visually or by instrumentation.

In solution hybridization procedures, the target nucleic acid and the probe(s) are brought together in solution and allowed to hybridize. Hybridization of the probe to the target can be followed by changes in optical density as double stranded nucleic acid is formed. Alternatively, double stranded nucleic acid may be separated from unreacted single strands and detected by means of a label present in the hybridized material. For example, hydroxyapatite can be used to selectively bind double stranded nucleic acids or S1 nuclease can be used to specifically degrade unreacted single stranded nucleic acid, which can then be separated from hybridized double stranded nucleic acid by size separation methods known in the art.

Solution hybridization is rapid and allows for efficient reaction between complementary nucleic acid sequences. However, subsequent separation and detection of the hybridized products is labor intensive, time consuming and difficult to automate. In an attempt to overcome some of these drawbacks, solid-surface hybridization methods have been developed in which one of the single stranded components of the hybridization mixture is immobilized on a solid support. Hybridization of the immobilized nucleic acid with complementary single stranded sequences in a solution of the remaining assay components results in immobilization of the entire hybridized complex through base pairing. The solid support is then easily removed from the solution to separate hybridized from unhybridized components. The occurrence of hybridization can be determined directly on the solid surface by detection of the label present in the hybrid.

Sandwich nucleic acid hybridization has improved the solid-surface hybridization methods by enhancing sensitivity. In these methods, at least two probes which do not hybridize to each other are hybridized to a single target nucleic acid. In one embodiment, one of the nucleic acid components of the hybridization mixture, usually either the target or an unlabeled probe, is immobilized on a solid support. The other probe carries the detectable label for detection of hybrid formation on the solid support.

In an alternative method of sandwich hybridization, one probe carries the detectable label and the other carries a second label (capture label) for binding the hybridized sandwich complex to the solid support. In this method, hybridization of the probes and the target usually takes place in solution and the complex is captured on the solid surface by binding of the capture label to a complementary capture ligand immobilized thereon. This type of sandwich hybridization is described in WO 88/02785, WO 86/07387, EP 425,217, EP 238,332 and GB 2,169,403.

Solid surface nucleic acid hybridization methods such as those described above provide rapid and simple separation of hybridized nucleic acids from unhybridized single stranded nucleic acids in the assay. However, they also exhibit several drawbacks. The kinetics of hybridization on a solid surface where not all of the reaction components can diffuse result in significantly slower reaction rates. As a result, hybridization and washing steps take much longer when a solid support is involved and assay sensitivity is often reduced. In addition, binding of nucleic acids to a solid support makes some of the bases unavailable for pairing, further reducing hybridization efficiency.

WO 89/10979 discloses a nucleic acid hybridization assay which attempts to reduce the time involved in performing the assay on a solid surface. In one embodiment, a sandwich hybridization is performed in solution and the hybrids are captured by a complementary capture ligand on a porous membrane in a flow-through membrane format as is commonly used for immunoassays. However, to achieve sufficient binding the solution is incubated with the membrane for 10 min. prior to allowing the solution to flow through. The sensitivity of the assay is not reported, as the target DNA was amplified by PCR prior to hybridization, thereby overcoming any problems of low sensitivity by providing increased amounts of target for detection.

Even the sandwich hybridization methods disclosed in the patent publications listed above suffer from the problem of long hybridization and washing times, although speed of separation is somewhat improved by having a complementary capture ligand on the solid surface rather than a hybridizing nucleic acid. In addition, some time savings have been achieved by using a controlled or restricted flow-through assay as in WO 89/10979. However, none of the prior art methods have found a way to take full advantage of the speed and simplicity of flow-through membrane assays for nucleic acid hybridization. That is, the prior art has been unsuccessful at eliminating a period of incubation with the membrane while maintaining a satisfactory level of sensitivity in nucleic acid hybridization assays.

The present invention overcomes these problems in the art of solid-surface nucleic acid hybridization. A flow-through membrane hybridization assay is provided which allows rapid, simple hybridization of nucleic acids and capture of the hybrids on a solid surface for detection. Applicants have discovered that specific combinations of membrane, buffers, and methods for immobilizing the complementary binding partner on the membrane allow performance of a flow-through membrane nucleic acid hybridization assay without restricting flow-through and without the requirement for any significant incubation time with the membrane. In addition to being more rapid than prior art assays of this type, sensitivity of the assay is improved.

### SUMMARY OF THE INVENTION

Applicants have unexpectedly discovered that the combination of certain porous membranes, certain buffers and certain methods for immobilizing the complementary binding partner on the membrane provides a highly sensitive flow-through nucleic acid hybridization assay which can be performed without a flow- restricting incubation step.

This discovery significantly reduces the time involved in performing such assays. Sensitivity is not reduced and therefore additional steps such as amplification of target DNA may not be necessary to compensate for lowered sensitivity as in other rapid nucleic acid hybridization assays. These advantages are made possible by Applicants' discovery that a particular combination of the porous membrane and methods and materials for coating it with the complementary binding pair member provide unexpected advantages in a flow-through membrane nucleic acid hybridization assay. The inventive assay can be performed in about 40 min. and is capable of detecting less than 50 attomoles of target nucleic acid (3 X ₁₀⁷ molecules).

### DETAILED DESCRIPTION OF THE INVENTION

According to the invention, single stranded target nucleic acid is hybridized in solution with one or more detector probes and one or more capture probes. The detector probe(s) contain a detectable label and the capture probe(s) contain a capture label which is a member of a specific binding pair. The capture label is capable of specifically binding to a capture ligand which is its complementary binding pair member. Solution hybridization is followed by immobilization of the probe:target complex on a porous membrane coated with the capture ligand. This is accomplished by passing the hybridization solution through the membrane in a flow-through membrane assay format.

The preferred flow-through membrane assay devices which are useful to perform the nucleic acid hybridization assays of the invention are essentially those previously known in the art for use in flow-through immunoassays. Appropriate devices for flow-through immunoassays are described in U.S. Patent No. 4,366,241, U.S. Patent No. 4,632,901, U.S. Patent No. 4,818,677 and U.S. Patent No. 4,920,046. The principles of operation of these devices remain the same when adapted to the inventive assay procedures, however, some modifications in the reagents and protocols are necessary, as disclosed below. Some of the flow-through assay devices known in the art describe a flow controlling layer which modulates the flow of solutions through the reaction membrane but does not prevent it. As used herein, the term "unrestricted flow-through" and related descriptions are intended to encompass and include flow controlling or modulating devices in which flow-through is regulated but not prevented. The invention is distinguished from prior art flow-through nucleic acid hybridization assays in that there is no period of incubation of assay components with the membrane during which flow-through is completely prevented until flow-through is desired.

To achieve the desired sensitivity with essentially immediate, unrestricted flow of the hybridization solution through the porous membrane, selection of the membrane material and buffers and the methods for coating it with the capture ligand are critical. Nylon membranes activated for covalent attachment of proteins provide the required characteristics. Streptavidin is bound to the membrane as a capture ligand by application of 5-100 I.Lg in a small volume of buffer to obtain a concentrated area of immobilized streptavidin. After capture of the probe:target complex and free capture probe on the membrane, unbound (i.e., unhybridized) detector probe is washed through the membrane with a wash buffer. After capture, the presence of the detector probe on the membrane is detected by means of its label. This indicates that probe:target complex has been bound.

The selection of the porous membrane is critical to achieve sufficient sensitivity on essentially immediate and unrestricted flow-through. For this purpose, nylon membranes activated for covalent attachment of proteins are suitable, whereas underivatized nylon membranes are not. The most preferred derivatized nylon membrane for use in the invention is the 0.45 µm IMMUNODYNE membrane (Pall BioSupport Division, Glen Cove, New York). In contrast, Pall's BIODYNE B and BIODYNE C nylon membranes, which have surfaces modified for positive and negative zeta potential only, failed to provide the desired specificity and sensitivity with unrestricted flow through the membrane. This is particularly unexpected, as the BIODYNE membranes are customarily used for nucleic acid binding assays and were developed for that purpose.

The quantity of capture ligand immobilized on the surface of the membrane and the materials and methods used to prepare it are critical for the high sensitivity of the assay. Many complementary binding pairs (i.e., capture labels and capture ligands) are known in the art and are used routinely in a variety of binding assays. Examples include antigens and antibodies, lectins and carbohydrates, enzymes and substrates, biotin and avidin, and biotin and streptavidin. Applicants have found that streptavidin provides the required characteristics for use as a capture ligand in the assay. Avidin, although similar to streptavidin, is not suitable for use in the assay. Unexpectedly, the desired properties for the complementary binding pair member are achieved only when streptavidin is immobilized on an activated nylon membrane such as IMMUNODYNE, but not when immobilized on other membrane types.

The specific coating conditions discovered further improve assay sensitivity. Preferably, the streptavidin is formulated at high concentration (about 10 mg/ml) and applied to the membrane in a small volume (about 1 - 10 µL). Preferably, about 5-100 I.Lg, most preferably about 50 I.Lg, of streptavidin is applied to a small area of the membrane. Most preferably, in order to further improve the level of sensitivity, the streptavidin is formulated in a high ionic strength buffer such as 1.0 M potassium phosphate, pH 7 for application to the membrane. Optimization of the stringency wash after binding of the probe:target complex can further improve the sensitivity of the assay. A room temperature wash solution of 10 mM sodium phosphate pH 7, containing 0.1% BSA and 0.1 % sodium dodecyl sulfate is preferred.

The detector probes of the invention are DNA oligonucleotides labeled with a detectable moiety which facilitates identification of the hybridized probe in the probe:target complex. The detector probe includes a sequence which is sufficient in length and sufficiently complementary to a sequence in the target nucleic acid to allow hybridization. The label may be directly detectable, or it may be rendered detectable after further reaction or manipulation. For example, the label may be a radioisotope (e.g., ^{32p} or ³H), a fluorescent dye (e.g., fluorescein) or an enzyme which is capable of reacting to form a colored or fluorescent product. Enzymes are preferred, most particularly alkaline phosphatase or horse radish peroxidase. The detector probe may be chemically synthesized with a defined nucleotide sequence, it may be isolated from DNA such as plasmids or genomic DNA, or it may be produced by transcription of DNA or RNA templates having a desired sequence. The label may be incorporated during synthesis or transcription (e.g. as described in U.S. Patent No. 4,711,955, European Patent Application No. 63879 and Maniatis et al. Molecular Cloning: A Laboratory Manual, Cold Spring Harbor Laboratories) or the label may be added to the completed detector probe using methods known in the art (e.g., as described in GB 2,153,356; Li et al. 1987. Nucleic Acids Research. 15: 5275; Jablonski et al. 1986. Nucleic Acids Research. 14: 6115). The foregoing methods and other are well known in the art and many are summarized in European Patent Application 238332, the disclosure of which is hereby incorporated by reference.

The capture probes of the invention are DNA oligonucleotides carrying biotin moieties to facilitate capture of the probe:target complex by the streptavidin capture ligand on the membrane. The capture probe also contains a sequence capable of hybridizing to a sequence in the target, but the hybridizing sequence is sufficiently distinct from that of the detector probe that hybridization of one probe does not significantly interfere with hybridization of the other. As for the detector probes, the capture probes may be chemically synthesized, transcribed from DNA or RNA templates or isolated from larger DNA sequences such as plasmids or genomic DNA using methods known in the art. If synthesized or transcribed, biotin may be incorporated by including biotinylated nucleotides in the transcription or synthesis (see U.S. Patent No. 4,711,955). Alternatively, biotin may be added to the completed capture probe using methods known in the art. Typically, the oligonucleotide probes are labeled with at least three biotin groups. When end-labeled probes are used, it has been noted that capture is improved by adding two to three biotin end-labels rather than the customary single biotin group.

The target nucleic acid sequence will usually be DNA but may also be RNA and the size will vary depending on the source and treatment of the target. The target nucleic acid contains sequences which are sufficiently complementary to sequences in both the detector probe(s) and the capture probe(s) to allow hybridization of both probes without significant interference. The target nucleic acid may be the genomic nucleic acid of a eukaryotic organism, a bacterial nucleic acid, a viral nucleic acid, a recombinant vector and the like. When large nucleic acid sequences are used as the target, they are usually made smaller by restriction or sonication prior to hybridization for ease of handling and manipulation. If it is not already single stranded, the target nucleic acid is made single stranded (denatured) prior to hybridization with the probes. This is customarily done by heating for 2-5 min. at about 95 °C, but other physical, chemical or enzymatic means known in the art can be used as well.

To perform the hybridization, the capture and detector probes are mixed with the denatured target nucleic acid in a hybridization buffer. Preferably the probes will be present in a concentration of about 20 nM, but the concentration may be adjusted within a range of 1 - 100 nM as needed. Any of the nucleic acid hybridization buffers known in the art may be used. The preferred hybridization buffer is 0.5 M sodium phosphate pH 7.0, 0.1% bovine serum albumin (BSA) and 0.1% sodium dodecyl sulfate (SDS). The hybridization is typically complete after about 5 min. at 50 _{°} C but the temperature may be varied between about 20-65 _{°} C with appropriate adjustments to hybridization time.

Applicants have found that using a 1 Kb target DNA and standard hybridization buffer components, as described above, the rate of hybridization is extremely slow below 50 _{°} C and is therefore not easily measured at room temperature. However, special conditions which allow improved hybridization of larger targets at room temperature have been discovered which typically result in only about a two-fold decrease in the signal from the detectable label as compared to the optimized 50 _{°} C hybridization. Three conditions for satisfactory room temperature hybridization have been found. These involve addition of any one of the following to the standard hybridization buffer prior to hybridisation: 1) 10-50% v/v formamide, preferably 35% formamide, 2) 10-50% w/v sodium trichloroacetate, preferably 25% sodium trichloroacetate, or 3) 10-50% v/v sodium trifluoroacetate, preferably 25% sodium trifluoroacetate.

Following hybridization, the entire hybridization mixture is applied to a streptavidin coated membrane prepared as described above. The liquid may be wicked through the membrane by contacting the membrane with an absorbent material, it may be drawn through the membrane by vacuum or it may be forced through by pressure. The preferred method is to wick the liquid through the porous membrane since this method does not require any additional equipment such as vacuums or pumps. Devices which wick liquid through the porous membrane are described in the U.S. Patent documents listed above, and any of these may be used in the practice of the invention. Most preferred is the device disclosed in U.S. Patent No. 4,920,046, which is sold for various immunoassay applications under the name COLORPAK by Becton, Dickinson and Company.

No period of incubation with the membrane prior to initiating flow-through is required, and the hybridization liquid is allowed to flow through the membrane essentially as soon as it is applied. As the liquid flows through the porous membrane, the unhybridized capture probe and the hybridized probe:target complex become bound to the streptavidin coated membrane through the biotin present in the capture probe. Excess unbound detector probe is drawn through the membrane by application of a room temperature wash solution. The preferred wash solution contains 10 mM sodium phosphate pH 7, 0.1% BSA and 0.1% SDS, but other wash solutions known in the art for nucleic acid binding assays are also suitable.

The label on the detector probe is then detected on the membrane. If the label is a fluorescent dye, light of the appropriate wavelength is applied and the presence or absence of fluorescence is observed and/or quantitated. If the label is a radioisotope, the radioactivity bound to the membrane is measured, e.g., by scintillation counting. If the label is a chromogenic dye, the necessary reactants are applied to the membrane to produce the colored product. For the preferred enzymatic labels, additional reactants are applied to the membrane to cause the formation of a colored reaction product on the membrane. The foregoing procedures are well known in the art of nucleic acid hybridization assays and immunoassays. For example, in the case of the preferred alkaline phosphatase label, a filter sterilized solution of 0.33 mg/ml nitroblue tetrazolium and 0.02% sodium azide in sterile water may be applied, followed by a filter sterilized solution of 2 mg/ml 3-indoxyl phosphate, 50 mM 2-amino-2-methyl-1-propanol and 0.2% sodium azide pH 10.5. The color development reaction may be stopped after about 10 min. by application of a 0.15 M citric acid stop solution, pH 2.

### EXAMPLE 1

### COMPARISON OF MEMBRANES

Five membranes were compared for their performance in the flow through nucleic acid hybridization assay using 5 fmole of DNA encoding the major outer membrane protein of Chlamydia trachomatis (MOMP) as the target nucleic acid. The membranes were IMMUNODYNE (nylon activated for covalent attachment, Pall Corp., Glen Cove, NY), BIODYNE (nylon modified for positive zeta potential, Pall Corp.), BIODYNE C (nylon modified for negative zeta potential, Pall Corp.), ULTRABIND (polysulfone membrane activated for covalent binding, Gelman Sciences Ltd., Northampton, UK) and nitrocellulose (Schleicher & Schuell, Inc., Keene, NH).

Four of each membrane type were spotted with 10 µL of 10 mg/ml streptavidin in phosphate buffered saline (PBS) pH 8.0, producing a small test area containing 100 µg of streptavidin. The membranes were dried 30 min. by vacuum and blocked for 30 min. with 0.3% casein, 0.05% NP-40 in PBS, pH 8. They were rinsed three times with PBS and dried again for 30 min. by vacuum. The prepared membranes were then assembled into flow-through devices similar to the COLORPAK assay device (Becton, Dickinson and Company).

For each assay, 50 µL of sterile water containing 5 fmole of the Chlamydia trachomatis major outer membrane protein (MOMP) DNA sequence and 5 µg salmon sperm carrier DNA was heated for 5 min. at 95 °C. The denatured DNA was cooled on ice, then 50 µL was added to the probe mixture. The probe mixture contained 5 pmole of biotin labeled capture probe (LC-BIOT-72), 5 pmole of alkaline phosphatase labeled detector probe (AP-223), 0.5% SDS and 1X hybridization buffer (0.5M NaP0₄ pH 7, 0.1% SDS and 0.1 % BSA). The hybridization mixture was incubated for 8 min. at 50 ° C.

The capture probe LC-BIOT-72 is a 21 base synthetic oligonucleotide with a long-chain biotin moiety (Pierce Chemical Co., Rockford, IL) attached to an amine functional group (AMINOLINK-2, Applied Biosystems, Ramsey, NJ) at the 5' end of the sequence. The base sequence corresponds to serovar L2 Chlamydia trachomatis MOMP sequence from position 1076-1096 (SEQ ID NO:1). the oligonucleotide with the 5' AMINOLINK-2 group was synthesized on an Applied Biosystems DNA synthesizer and purified by denaturing acrylamide gel electrophoresis and ethanol precipitation. The purified AMINOLINK-2 oligonucleotide was then chemically linked to LC-biotin and the biotinylated oligonucleotide was purified by conventional procedures using reverse phase HPLC. The completed capture probe has the following structure:

The detector probe AP-223 is a 21 base synthetic oligonucleotide that was conjugated through a 5' amine group (AMINOLINK-2) to alkaline phosphatase using conventional procedures (Jablonski, E., et al. Nucleic Acids. Res. 14:6116-6128 (1986)). The base sequence corresponds to serovar L2 Chlamydia trachomatis MOMP sequence from position 301-321 (SEQ ID NO:2). The completed detector probe has the following structure:

The prepared membranes in the flow-through devices were prewet with 200 µL PBS, pH 8 prior to placing the triangle insert over the membrane. 100 µL of the hybridization mixture was applied through the insert and the insert was removed. The membrane was washed four times with 200 µL of wash buffer (10mM NaP04 pH 7, 5X Denhardt's solution^{*}and 0.1% SDS. This was followed by two washes with 200 µL 0.5X SSC-and one wash with 300 µL IX detection buffer (100 mM Tris-HCI pH 8.8, 100 mM NaCI, 5 mM MgC1₂).

Two hundred µL of the enzyme substrate composition, containing a 1:1000 dilution of stock nitroblue tetrazolium solution (75 mg/ml in 70% dimethylfromamide) into 1X detection buffer (0.15 mg/ml 5-bromo-4-chloro-dimethylformamide) was added to the membrane and allowed to flow through. After 10 min. another 200 µL aliquot of substrate was applied, followed by a third 200 µL aliquot after an additional 10 min. At that time the color reaction was stopped with 200 µL of 0.15M citric acid, pH 2.

The expected positive reaction was a blue to purple triangle visible on the surface of the membrane. Only the IMMUNODYNE membrane developed a positive signal. The BIODYNE C membrane showed a ^{*}0.1 % BSA, 0.1% FICOLL 400, 0.1% polyvinylpyrrolidone ^{**} 20X SSC = 3.0M NaCl, 0.3M sodium citrate, pH 7.0 white triangle with a purple outline. The remaining membranes were negative.

In a similar experiment, the IMMUNODYNE membrane was compared with the IMMOBILON-AV membrane (a polyvinylidene difluoride [PVDF] membrane modified for covalent attachment, Millipore, Bedford, MA). Four membranes were prepared for each test - a) IMMOBILON with streptavidin applied in PBS, pH 8, b) IMMOBILON with streptavidin applied in 0.5M K₂HP0₄ pH 7.4 with 1 mg/ml dimethylamino pyridine (DMAP), c) IMMUNODYNE with streptavidin applied in PBS, pH 8, and d) IMMUNODYNE with streptavidin applied in 0.5M K2 HP0₄ pH 7.4 with 1 mg/ml DMAP. For each membrane, 5 u. L of a 10 mg/ml solution of streptavidin in the appropriate buffer as described above was applied to a small area, resulting in a test site containing 50 µg of streptavidin. After about 10 min. incubation, the membranes were dried, blocked, rinsed and dried as in the previous experiment.

For each assay, 50 µL of sterile water containing 5 fmole of MOMP DNA and 5 µg of salmon sperm DNA was heated for 5 min. at 95 °C. The denatured samples were cooled on ice and then 50 µL of denatured target DNA was added to 50 µL of the probe mixture. The probe mixture was as in the previous experiment, but with SDS reduced to 0.1 %. The hybridization mixture was added to the devices and the membranes were washed as in the previous experiment. To develop the color reaction, 150 µL of substrate A (0.33 mg/ml nitroblue tetrazolium, 0.02% sodium azide in sterile water) was added followed by 150 µL of substrate B (2 mg/ml 3-indoxyl phosphate, 50mM 2-amino-2-methyl-1-propanol, 0.2% sodium azide, pH 10.5). Color development was stopped after 10 min. by application of 0.15M citric acid pH 2.

No positive reaction was seen on the IMMOBILON membranes but the IMMUNODYNE membranes developed a blue-purple triangle indicating a positive reaction. Signal strength was significantly better when the streptavidin was applied in the 0.5M K₂HPO₄,pH 7.4/1 mg/ml DMAP buffer.

### EXAMPLE 2

### DETECTION OF A 99 BP TARGET NUCLEIC ACID SEQUENCE

IMMUNODYNE membranes were prepared by slow application of 5 µL of a 10 mg/ml streptavidin solution in 1.0M potassium phosphate pH 7.4 to produce a small test area containing 50 µg of streptavidin. The membranes were allowed to dry for more than 10 min. and then were blocked, rinsed and dried as described in example 1. Preparations of a 99-mer MOMP DNA fragment (positions 718-816) were made in sterile water such that the following quantities were contained per 10 µL:
0.5 fmole MOMP + 5 µg salmon sperm DNA
0.2 fmole MOMP + 5 µg salmon sperm DNA
0.1 fmole MOMP + 5 µg salmon sperm DNA
0.05 fmole MOMP + 5 µg salmon sperm DNA
0.025 fmole MOMP + 5 µg salmon sperm DNA

Each target level was assayed in duplicate. The target DNA's were denatured for 3 min. at 95 _{°} C and cooled on ice. Ninety µL of the probe mixture was then added per 10 mL of the probe mixture was then added per 10 µL of denatured target for a 100 µL hybridization reaction. The hybridization reaction contained 2 pmole biotin labeled capture probe (CT248-LCB(2-4)) 1 pmole alkaline phosphatase labeled detector probe (CT246-AP) 0.5M NaP0₄, 0.1% BSA and 0.1% SDS in sterile water. The hybridization reaction was incubated fro 5 min. at 50 °C. The prepared membranes in flow-through devices were prewet with 100 µL of water and then 100 µL of the hybridization reaction was applied through the triangle insert and the insert was removed. The membrane was washed three times with 300 µL of wash buffer containing 10mM sodium phosphate pH 7.0, 5X Denhardts solution and 0.1% SDS, and once with 300 µL of 1X detection buffer. As before, 150 µL of substrate A followed by 150 µL of substrate B was added to develop the color reaction. After 10 min. color development was stopped by addition of 300 µL of citrate stop solution. A similar experiment was performed in which a second biotin labeled capture probe, CT249-LCB-(2-4), was substituted for CT248-LCB(2-4).

The capture probe CT-248-LCB(2-4) is a 29 base synthetic oligonucleotide with a mixture of 2-4 long-chain biotin moieties (Pierce Chemical Co.) attached through amine functional groups (AMINO MODIFIER II, Clontech Laboratories, Inc., Palo Alto, CA) at the 5' end of the sequence. The oligonucleotide with four amine functional groups at the 5' end was synthesized on an Applied Biosystems DNA synthesizer and purified by denaturing acrylamide gel electrophoresis and ethanol precipitation. The purified multi-amine oligonucleotide was then chemically linked to LC-biotin and the multibiotinylated oligonucleotide (2-4) was purified by conventional procedures using reverse phase HPLC. The sequence corresponds to serovar L2 Chlamydia trachomatis MOMP sequence from position 718-746 (SEQ ID NO:3). The completed probe has the following structure:

The capture probe CT-249-LCB(2-4) is a 20 base synthetic oligonucleotide prepared as described above, with a mixture of 2-4 long-chain biotin moieties attached to the 5' end. The sequence corresponds to serovar L2 Chlamydia trachomatis MOMP sequence from position 747-766 (SEQ ID NO:4). The completed probe has the following structure:

The detector probe CT-246-AP is a 22 base synthetic oligonucleotide that was conjugated through a 5' amine group (AMINOLINK-2, Applied Biosystems) to alkaline phosphatase as described above. The sequence corresponds to serovar L2 Chlamydia trachomatis MOMP sequence from position 767-788 (SEQ ID NO:5). The completed probe has the following structure:

The membranes were scanned with a Greytag Densitometer, set for B reading and blanked on the outside circle of the membrane. A positive signal was detected at the lowest concentration of target nucleic acid tested, i.e., 25 attomoles. Similar assay sensitivity was achieved with either capture probe indicating that there was no stearic hindrance with the detector probe in either case.

The experiment was repeated using 100, 50, 25 and 10 attomole of target MOMP DNA. Using the CT248-LCB(2-4) capture probe, 10 attomoles of target were weakly detected and the 0 attomole MOMP control was negative. However, using the CT249-LCB(2-4) capture probe, 10 attomoles could be weakly detected but the 0 MOMP control was also weakly positive. This suggests that CT249-LCB(2-4) has slightly different properties is a capture probe. Using the capture probes in combination did not significantly increase the sensitivity for detection of the 99 bp target DNA.

### EXAMPLE 3

### DETECTION OF TARGET DNA WITH MULTIPLE CAPTURE AND DETECTOR PROBES

Two biotin labeled capture probes and three alkaline phosphatase labeled detector probes were used to detect a 1 Kb MOMP target DNA sequence. IMMUNODYNE membranes were prepared as described in Example 2. Duplicate tests were run with three-fold dilutions of MOMP DNA covering a range of concentrations from 3,000 attomoles to 4 attomoles per assay diluted in 50 µg/ml salmon sperm DNA carrier. Triplicate controls were run with no target DNA added.

Target DNA in a volulme of 30 /1.L per assay was denatured for 3 min. at 95 _{°} C and put on ice. A probe reagent mixture was added to each target sample to give a 100 /1.L hybridization reaction containing 10 pmole of each biotin labeled capture probe (CT-48-LC BIOT(2) & CT-72-LC-BIOT(2) described below), 5 pmole each of two alkaline phosphatase labeled detector probes (CT-27(a)-AP & CT-247-AP, described below), 1 pmole of one alkaline phosphatase labeled probe (CT-246-AP, described below), 0.1% SDS, 4X SSC and 5X Denhardts solution. The mixture was allowed to hybridize for 5 min. at 50 C. The prepared membranes in flow-through devices were prewet with 200 µL of PBS and then 100 µL of hybridization mixture was applied through the triangle insert and the insert was removed. The membranes were washed, the substrates applied for a 10 minute incubation and color develeopment stopped as described in Example 2.

A visual reading after 10 min. showed a faint blue triangle with as little as 12 attomoles of target. A clear signal was seen in less than 5 min. for 37 attomoles of target nucleic acid. It is expected that using a dual enzyme detection system such as that disclosed by Mize et al. in U.S. Patent No. 4,835,099 would further increase assay sensitivity 10-30 fold.

Two capture probes were used in this example. Capture probe CT-48-LC-Biotin(2) is a 26 base synthetic oligonucleotide corresponding to serovar L2 Chlamydia trachomatis MOMP sequence from position 1216-1241 (SEQ ID NO:6) At the 5' end of the sequence four amine functional groups were attached during the oligo synthesis using the branched modifier C3 reagent from Glen Research Corp. (Sterling, VA). Long-chain biotin moieties (Pierce Chemical Co.) were attached through theh amine groups and the biotinylated oligonucleotide was purified as described previously. The completed probe has the following structure:

The second capture probe, CT-72-LC-Biotin(2), was prepared in the same manner and is a 21 base synthetic oligonucleotide corresponding to serovar L2 Chlamydia trachomatis MOMP sequence from position 1076-1096 (SEQ ID NO:7). The completed probe has the following structure:

The three detector probes used in this example were prepared as described previously and have alkaline phosphatase attached through a 5' amino group (AMINOLINK-2). CT-27a-AP is a 21 base synthetic oligonucleotide corresponding to serovar L2 Chlamydia trachomatis MOMP sequence from position 301-321 (SEQ ID NO:8). This probe has the structure AP-AL2-5'-GGAGATCCTTGCGATCCTTGC-3'. CT-247-AP is a 23 base synthetic oligonucleotide corresponding to position 544-566 (SEQ ID NO:9). This probe has the structure AP-AL2-5'-GCATTGAATATTTGGGATCGTTT-3'. CT-246-AP is a 22 base synthetic oligonucleotide corresponding to position 767-788 of the MOMP sequence (SEQ ID NO:10). Its structure is AP-AL2-5'-

## Claims

1. A method for detecting a target nucleic acid in a sample comprising:
a) hybridizing the target nucleic acid in solution with at least one detector probe including a detectable label and at least one capture probe including a biotin label such that a probe:target complex is formed;
b) applying the solution containing the probe:target complex to a porous membrane comprising nylon activated for covalent attachment of proteins, the membrane having thereon 5-100µg of streptavidin in a test site;
c) allowing unrestricted flow of the solution through the membrane such that the probe:target complex becomes bound to the membrane at the test site, and;
d) detecting, by means of the detectable label, the probe:target complex bound to the membrane.

2. The method of Claim 1 wherein the complex is detected by means of an enzyme label which is reacted to form a colored reaction product.

3. The method of Claim 2 wherein the membrane has about 50µg of streptavidin in the test site.

4. The method of Claim 1 or 2 wherein the capture probe, detector probe and target nucleic acid are hybridized at room temperature.

5. The method of Claim 4 wherein the capture probe, detector probe and target nucleic acid are hybridized in a buffer including an ingredient selected from the group consisting of 10-50% v/v formamide, 10-50% w/v sodium trichloroacetate and 10-50% v/v sodium trifluoroacetate.

6. The method of Claim 5 wherein the buffer includes an ingredient selected from the group consisting of 35% v/v formamide, 25% w/v sodium trichloroacetate and 25% v/v sodium trifluoroacetate.

7. A method for detecting a target DNA in a sample comprising:
a) hybridizing the target DNA in solution with at least one detector probe including an enzyme label which can be reacted to form a colored product and at least one capture probe including a biotin label such that a probe:target DNA complex is formed;
b) applying the solution containing the probe:target DNA complex to a porous membrane comprising nylon activated for covalent attachment of proteins, the membrane having thereon 5-100µg of streptavidin applied to the membrane in about 1-10µL of 1 M potassium phosphate pH 7 buffer to form a test site;
c) causing unrestricted flow of the solution through the membrane by contacting the membrane with an absorbent material such that the probe:target complex becomes bound to the membrane at the test site, and;
d) detecting the probe:target complex bound to the membrane by reacting the enzyme label to produce a colored product.

8. The method of Claim 7 wherein the enzyme label is selected from the group consisting of alkaline phosphatase and horseradish peroxidase.

9. The method of Claim 7 wherein the streptavidin is applied to the membrane in about 5αL of buffer.

10. The method of Claim 13 wherein the enzyme reaction is stopped by addition of citric acid.
